# EUROPEAN PATENT SPECIFICATION

(11) **EP 2 832 723 B1**
(45) Date of publication and mention of the grant of the patent: **15.02.2017**
(21) Application number: 13003774.0
(22) Date of filing: 29.07.2013
(51) Int. Cl.: C07D 209/52, A61K 9/10

(54) **Stabilised amorphous forms of Saxagliptin**
Stabilisierte amorphe Formen von Saxagliptin
Formes amorphes stabilisées de la Saxagliptine

(43) Date of publication of application: 04.02.2015
(73) Proprietor: Zentiva, k.s., 102 37 Praha 10 (CZ)
(72) Inventor: Davis, Dita, Quinton, Birmingham B32 1EZ (GB); Ridvan, Ludek, 102 00 Praha 10 (CZ); Stepankova, Hana, 282 01 Cesky Brod (CZ); Sedmak, Gregor, 1291 Skofljica (SI)
(74) Representative: Jirotkova, Ivana

(56) References cited:
- EP-A1- 0 852 140
- EP-A1- 2 105 130
- EP-A2- 1 027 886
- WO-A1-2013/136343
- US-A1- 2012 083 517
- US-B2- 8 257 739
- BABAR IQBAL ET AL: "Recent Advances and Patents in Solid Dispersion Technology", RECENT PATENTS ON DRUG DELIVERY & FORMULATION, vol. 5, no. 3, 1 September 2011 (2011-09-01), pages 244-264, XP055023758, ISSN: 1872-2113, DOI: 10.2174/187221111797200551

## Description

### Technical Field

The present invention relates to novel stabilised amorphous forms of Saxagliptin, methods of preparation as well as use in pharmaceutical compositions.

### Background Art

The compound (1S,3S,5S)-2-[(2S)-2-amino-2-(3-hydroxy-1-adamantyl)acetyl]-2-azabicyclo[3.1.0]hexane-3-carbonitrile (Saxagliptin) of formula 1 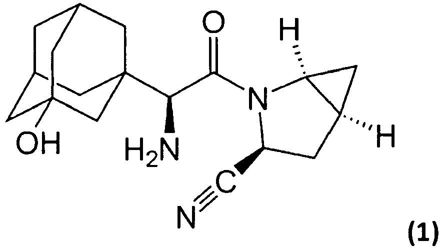 is known to be a new oral hypoglycemic (anti-diabetic drug) of the new dipeptidyl peptidase-4 (DPP-4) inhibitor class of drugs. It is a therapeutic agent for treatment of Type-2 diabetes mellitus, obesity and related diseases as shown in U.S. Patent No.6,395,767, example 60.

As has been described in WO2008/131149 and US2012/0083517, Saxagliptin can exist as hydrate, solvate, free base or pharmaceutically acceptable salts. Also amorphous form of Saxagliptin was described as well as process for preparation of individual forms and use thereof in pharmaceutical formulation. For preparation of pharmaceutical composition however, predominantly crystalline forms of Saxagliptin are used due to the lower stability and usually higher content of impurities present in amorphous form.

### Subject matter of the invention

The invention is defined in the appended claims and involves glass solution of saxagliptin base, or pharmaceutically acceptable salt thereof, dispersed within a polymer matrix, wherein the polymer is selected from hydroxypropyl methylcellulose and hydroxypropylcellulose, process for its the preparation and its use for the preparation of the pharmaceutical composition.

### Detailed description of the invention

The present invention relates to the solid state structures of (1S,3S,5S)-2-[(2S)-2-amino-2-(3-hydroxy-1-adamantyl)acetyl]-2-azabicyclo[3.1.0]hexane-3-carbonitrile (hereafter referred to as Saxagliptin) in the form of a stable amorphs and their preparation.

Crystalline solid is characterised by its long-range order. On the other hand, amorphous solids lack the long-range order. The molecular arrangement in the amorphous solid may be represented by a frozen liquid with the rheological properties of a solid.

As used herein, the term "solid dispersion" means any solid composition having at least two components. In certain embodiments, a solid dispersion as disclosed herein includes an active pharmaceutical ingredients -API (saxagliptin) dispersed among at least one other component, for example a polymer.

The term "molecularly dispersed", as used herein, refers to the random distribution of a compound (e.g. saxagliptin) with a polymer. In some embodiments, saxagliptin may be dispersed within a matrix formed by the polymer in its solid state such that the saxagliptin is immobilised in its amorphous form. When saxagliptin is molecularly dispersed in a polymer the resulting solid dispersion is having a single glass transition temperature (Tg) and its herein referred to as a glass solution.

On the other hand if a compound (e.g. saxagliptin) is transformed into an amorphous state but is not molecularly dispersed in a polymer matrix because amorphous saxagliptin clusters are incorporated in a polymer instead, the solid dispersion used herein is called a glass suspension. The resulting glass suspension having two glass transition temperatures; one results from the amorphous API and the other one results from a polymer.

As it is known in the art, because of the lack of a long-range order and usually high surface area, amorphous solids typically exhibit higher solubility than crystalline form. In order to increase solubility of a crystalline solid, it is thus beneficial to prepare active pharmaceutical ingredient in an amorphous form.

If a crystalline material is heated until its melting point is reached (Tm), the material is transformed from solid to liquid state. This is reversible, so upon cooling, the molten liquid rearranges back to crystal lattice. If, however, the liquid is cooled sufficiently fast below its melting point, crystallisation may be prevented forming a super-cooled liquid. In the super-cooled liquid is cooled enough to reach glass transition temperature (Tg), the molecules are kinetically frozen and the super-cooled liquid solidifies into a glass. In general, molecules in a super-cooled liquid state have have much higher mobility as in a glassy state as described in Remington: The Science and Practice of Pharmacy, Pharmaceutical Press, 21nd edition.

Since the molecules even in a glassy state have some mobility, it is known in the art that it is beneficial that the glass transition temperature of active pharmaceutical ingredient is at least 20 °C higher, more preferably at least 30 °C higher and most preferably at least 40 °C higher than the actual storage conditions.

Amorphous Saxagliptin has relatively low glass transition temperature (Tg), which is about 54°C. Therefore, the amorphous form undergoes recrystalisation upon storage conditions. It is therefore highly beneficial to stabilise amorphous form of Saxagliptin by increasing the glass transition temperature (Tg) in order to prevent recrystallisation.

Amorphous form of Saxagliptin can be stabilised by mixing the API with a second component. There are a number of different second components that can be used. In general, all second components used to stabilise Saxagliptin have a common characteristic, which is that they decrease mobility of Saxagliptin molecules and thus prevent the recrystallisation.

One type of the second component used for stabilisation of amorphous Saxagliptin are polymers. Suitable polymers used may be selected from, but not restricted to water soluble as well as water insoluble polymers. Typical water soluble polymers suitable to stabilise amorphous Saxagliptin are polyvinylpyrrolidone (povidone), copovidone, polyvinyl alcohol, hydroxypropyl methylcellulose (hypromellose), hydroxypropylcellulose, polyvinyl caprolactam-polyvinyl acetate-polyethylene glycol graft co-polymer (Soluplus™), and similar. Typical water insoluble polymers suitable to stabilise amorphous Saxagliptin are methylcellulose, ethylcellulose, polymethacrylates, hypromellose phthalate, hypromellose acetate succinate, cellulose acetate phthalate, carboxymethyl ethyl cellulose and crospovidone.

According to the present invention there is provided a glass solution of saxagliptin base, or pharmaceutically acceptable salt thereof, dispersed within a polymer matrix, wherein the polymer is selected from hydroxypropyl methylcellulose and hydroxypropylcellulose, wherein the hydroxypropyl methylcellulose is with molecular weight about 22,000 Da (g/mol) and the hydroxypropylcellulose is with molecular weight about 80,000 Da (g/mol) and wherein the ratio of the amount by weight of saxagliptin within the glass solution to the amount by weight of the polymer therein is 1:2.

There exist a number of possible processes to prepare the stabilised amorphous form of Saxagliptin.

One possible process is the so-called solvent processes. What is common to all solvent processes is that the active ingredient is dissolved in a solvent or in any mixture thereof. Solvents can be water or any organic solvents. Some examples of possible organic solvents are methanol, ethanol, ethyl acetate, isopropanol, acetone, dicloromethane, tetrahydrofuran etc. In the next step, a second component to stabilise Saxagliptin is added forming either solution or suspension. The solvent is then quickly removed and a super-cooled glassy solid remains. Some concrete solvent processes used to prepare composition of Saxagliptin in stabilised amorphous form may be selected from, but not restricted to removal of a solvent in a rotary evaporator, fluid bed granulation, spray drying, electrospinning, freeze drying and similar.

The second possible process is the process without the use of a solvent. In this process, Saxagliptin is mixed directly with the second component to stabilise amorphous form and melted in order to form a melt. In general, the melt is heated to approximately 20-40 °C above the Tg of the melted system in order to decrease the viscosity of a system to such a degree that it is possible to process the system. The melt is subsequently cooled in order to form a super-cooled glassy solid. Some concrete examples of this process may be selected from, but not restricted to hot melt extrusion, hot melt granulation in a high shear mixer, solvent-free melt granulation in a fluid bed granulator and the like.

Also described is a solid pharmaceutical composition comprising Saxagliptin and at least one second component used to stabilise amorphous Saxagliptin, preferably polymer selected from a group comprising polyvinylpyrrolidone, hydroxypropyl methylcellulose and hydroxypropylcellulose, more preferably PVP K30, PVP K25, Methocel ™ E5 and
Klucel ™ EF polymers.

PVP K30 is polyvinylpyrrolidone polymer with molecular weight about 50,000 Da (g/mol).

PVP K25 is polyvinylpyrrolidone polymer with molecular weight about 30,000 Da (g/mol).

Methocel ™ E5 is hydroxypropyl methylcellulose polymer with molecular weight
about 22,000 Da (g/mol).

Klucel ™ EF is hydroxypropylcellulose polymer with molecular weight about 80,000 Da (g/mol).

Two approaches can be applied for preparation of glass solutions by spray drying technique. Saxagliptin can be used either as a salt or as a free base *in situ* with an acid, yielding Saxagliptin dispersed in a matrix formed by the polymer. The spray dried products were assessed by XRPD and DSC analysis and the results are summarised in Table 1.

The XRPD results demonstrated that Saxagliptin with PVP K30, Klucel ™ EF, Methocel ™

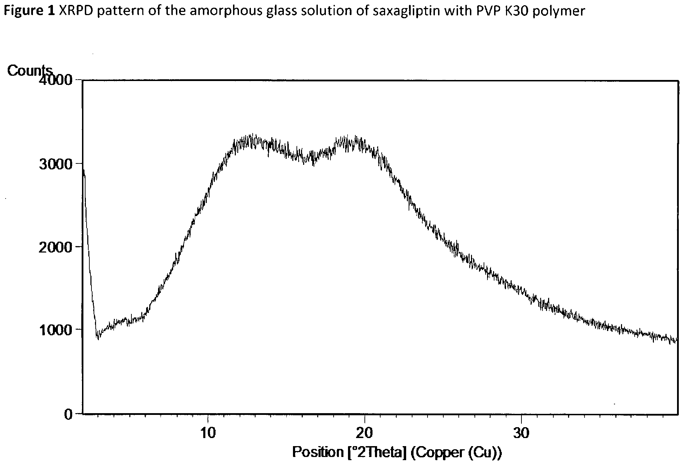

E5 and PVP K25 polymers formed amorphous solid dispersions. All diffractograms displayed a broad amorphous halo with the absence of sharp signals as shown in Figure 1 as a representative example.

In differential scanning calorimetry (DCS) measurements, solid dispersions of saxagliptin and polymer selected from PVP K30, Klucel ™ EF, Methocel ™ E5
exhibited a single glass transition temperature (Tg) indicating complete miscibility between the API and polymer.

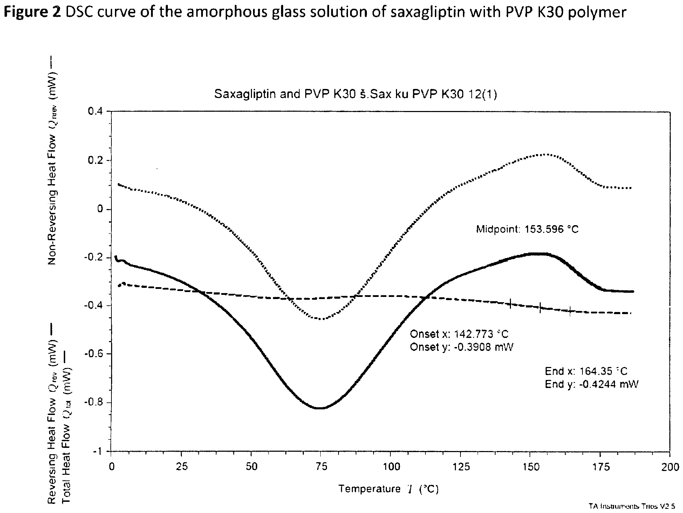

It is known that the physical stability of the glass solution is increased with a higher glass transition temperature (Hancock and Zografi, 1997). Comparing all recorded Tg values, the highest glass transition temperature was observed for the PVP K30 glass solution as shown in Figure 2.

Saxagliptin is a crystalline drug with a melting point at about 54 °C, while PVP K30 is amorphous solid with Tg value of 167 °C. Therefore, the observed single Tg value of 154°C for this solid dispersion is the indication that the glass solution is consisted of only one phase.

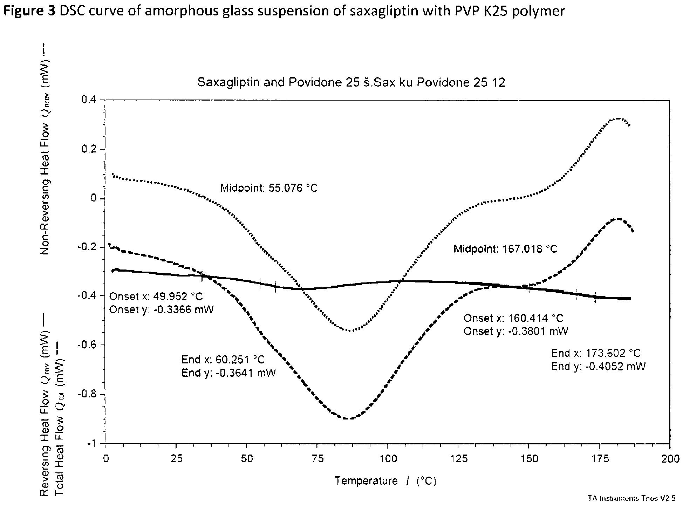

However, in the case of PVP K25 solid dispersion, the DSC curve displayed two glass transition temperatures as shown in Figure 3. One Tg at 55°C resulted from the amorphous API and the other Tg at 167°C resulted from the amorphous polymer PVP K25. This means that saxagliptin was transformed into an amorphous state but was not molecularly dispersed in the polymer matrix. Instead amorphous saxagliptin clusters were incorporated in PVP K25, yielding in formation of a glass suspension not the glass solution.

It was found out that a 1:2 weight/weight ratio of API to polymer was able to form physically stable glass solutions or suspensions.

The following solid dispersions with PVP K30 and PVP K25 are not encompassed by the claims.

**Table 1. Amorphous stability evaluation of some solid dispersions prepared by spray drying.**

| **Polymer** | **XRPD** | **DSC Tg (°C)** | **Results after open exposure at 40°C/75% RH** |
|---|---|---|---|
| PVP K30 | amorph | 154 | Stable after storage for up to 4 weeks |
| Klucel EF | amorph | 110 | Stable after storage for up to 4 weeks |
| Methocel E5 | amorph | 140 | Stable after storage for up to 4 weeks |
| PVP K25 | amorph | 55 & 167 | Stable after storage for up to 4 weeks |

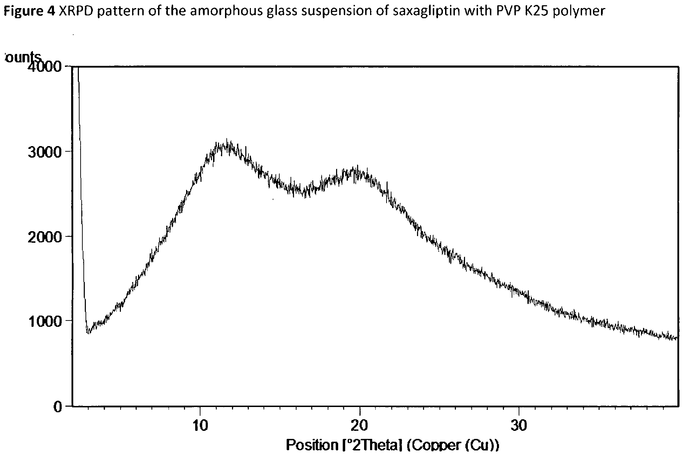

Solid dispersion prepared by hot melt extrusion process were also analysed by XRPD. Patterns of the X-ray powder diffraction exhibited a broad halo peak characteristic of amorphous glass solutions as shown in Figure 4 as a representative example. Non-occurance of sharp signals in all diffractograms, which could be allocated to the crystalline form of saxagliptin, confirmed that saxagliptin is molecularly dispersed within the polymers.

The amorphous solid dispersions were characterised by DSC measurements. The glass transition temperature were very similar to those obtained by spray drying technique. Again the highest glass transition temperature of 156 °C was observed for the matrix consist of saxagliptin and PVP K30 polymer. The presence of just one glass transition temperature confirmed that saxagliptin was successfully immobilised in its amorphous form in uniphase matrix as shown in Table 2.

**Table 2. Amorphous stability evaluation of some solid dispersions prepared by hot melt extrusion process.**

| **Polymer** | **XRPD** | **DSC Tg(°C)** | **Results after open exposure at 40°C/75% RH** |
|---|---|---|---|
| PVP K30 | amorph | 156 | Stable after storage for up to 4 weeks |
| Klucel EF | amorph | 114 | Stable after storage for up to 4 weeks |
| Methocel E5 | amorph | 142 | Stable after storage for up to 4 weeks |

The stored samples were evaluated by XRPD and the results did not indicate any crystalline peak in powder XRPD profiles after 4 weeks of open exposure at 40°C and 75% RH.

Glass solutions of saxagliptin within the polymer matrix according to this invention can be used in the preparation of pharmaceutical composition, especially solid composition, e.g. tablets. Such a solid composition can further comprise at least one pharmaceutical excipient selected from fillers (e.g. lactose), binders (e.g. microcrystalline cellulose), disintegrants (e.g. croscarmellose sodium), lubricants (e.g. MgSt), surfactants, etc. Tablet composition can be optionally coated by any conventional coating, such as polyvinylalcohol or polyethylene glycol.

### Brief description of the drawings

1. Figure 1 provides the XRPD pattern of the amorphous glass solution of saxagliptin with PVP K30 polymer (comparative).
2. Figure 2 provides the DSC curve of the amorphous glass solution of saxagliptin with PVP K30 polymer (comparative).
3. Figure 3 provides the DSC curve of amorphous glass suspension of saxagliptin with PVP K25 polymer (comparative).
4. Figure 4 provides the XRPD pattern of the amorphous glass suspension of saxagliptin with PVP K25 polymer (comparative).

### Characterisation methods and preparation methods

**DSC** curves were recorded using a Perkin Elmer Pyris 1 differential scanning calorimetr. For the measurements approximately 2 - 6 mg of sample were placed in aluminium pans, accurately weighed and hermatically closed with perforation lids. Prior to measurement the lids were pierced resulting in approximately 1.5 mm pin holes. The samples were then heated under a flow of nirtogen of about 100 mL/min using heating rates of usually 10 K/min.

**XRPD** patterns were obtained with laboratory X-Ray diffractometer X'PERT PRO MPD PANalytical operating in diffraction mode θ - θ with copper radiation CuKα (λ=1.542 Å, 45 kV/ 40 mA), in 2-theta range 2 - 40° 2θ, with step 0.02°2θ and step time 3 s. Samples were measured on Si plate (zero-background holder). Primary optics setting: Soller slits 0.02 rad, automatic PDS, 10 mm mask, 1/4° anti-scatter slit, irradiated sample area 10 mm. Secondary optics setting: 5.0 mm anti-scatter slit, Soller slits 0.02 rad, detector X'Celerator with maximal active length.

### Physical stability testing

Storage stability of amorphous products (saxagliptin dispersed within a polymer selected from PVP K30, Klucel ™ EF, Methocel ™ E5 and PVP K25) were carried out at
75% RH and at controlled temperature (40°C) for 7 days, then further for 4 weeks. The samples were evaluated by XRPD. The results are shown in the Tables 1 and 2.

### Spray drying

Saxagliptin and polymer were spray dried using Buchi B-290 spray-drier. The following conditions were used for all experiments; aspirator flow: 100%, gas flow rate: 577 l/min, solution flowrate: 1.0-2.0 ml/min, inlet temperature: 50°C.

### Hot melt-extruder

Saxagliptin and polymer blends were extruded using Three-Tec Extruder ZE 12 ZD 5 FB a twin-screw extruder with a temperature cascade rising to 165 °C.

### Examples

### Example 1- Spray drying

Saxagliptin free base (1 g), which was acidified with aq. HCl, and polymer (2 g) (selected from PVP K30, Klucel ™ EF, Methocel ™ E5 and PVP K25) was dissolved in
dichloromethane and ethanol solvent mixture (150 ml). The resulting solution was then spray dried using a Buchi B-290 spray drier. Obtained amorphous products were dried in a vacuum oven over desiccant at 40°C for 12 h.

### Example 2- Spray drying

Saxagliptin hydrochloride (1 g) and polymer (2 g) (selected from PVP K30, Klucel ™ EF, Methocel ™ E5 and PVP K25) were dissolved in mixture of dichloromethane
and ethanol (150 ml) then spray dried using a Buchi B-290 spray drier. The spray dried amorphous products were dried in a vacuum oven over desiccant at 40°C for 12 h.

### Example 3 - Hot Melt Extrusion

Saxagliptin hydrochloride (1 g) was mixed with polymer (2 g) (selected from PVP K30, Klucel ™ EF and Methocel ™ E5). The mixture was then fed continuously to a temperature-controlled extruder. The resulting extrudate was cooled to room temperature yielding in amorphous product.

## Claims

1. Glass solution of saxagliptin base, or pharmaceutically acceptable salt thereof, dispersed within a polymer matrix, wherein the polymer is selected from hydroxypropyl methylcellulose and hydroxypropylcellulose, wherein the hydroxypropyl methylcellulose is with molecular weight about 22,000 Da (g/mol) and the hydroxypropylcellulose is with molecular weight about 80,000 Da (g/mol) and wherein the ratio of the amount by weight of saxagliptin within the glass solution to the amount by weight of the polymer therein is 1:2.

2. Glass solution according to claim 1 that exhibits a powder x-ray diffraction pattern having characteristic of amorphous halo.

3. Glass solution according to any of the preceding claims **characterised in that** it is a single-phase with a glass transition temperature (Tg) of more than 100°C.

4. A process for the preparation of glass solution of saxagliptin, defined in any of claims 1-3, wherein saxagliptin base, or pharmaceutically acceptable salt thereof, and the polymer are dissolved in a solvent selected from organic solvents and water or mixture thereof and then spray dried.

5. The process according to claim 4 wherein the organic solvent is methanol, ethanol, ethyl acetate, isopropanol, acetone, dichloromethane, tetrahydrofuran or mixture thereof.

6. The process according to claim 5 wherein the solvent is mixture of DCM and ethanol.

7. A process for the preparation of glass solution of saxagliptin, defined in any of claims 1-3, wherein saxagliptin base, or pharmaceutically acceptable salt thereof, is mixed with polymer and the mixture is extruded by use of temperature-controlled extruder.

8. Use of the glass solution of saxagliptin within a polymer matrix according to any of the preceding claims for the preparation of a pharmaceutical composition.

## Patentansprüche

1. Glaslösung von Saxagliptinbase oder einem pharmazeutisch annehmbaren Salz davon, dispergiert in einer Polymermatrix, wobei das Polymer ausgewählt ist aus Hydroxypropylmethylcellulose und Hydroxypropylcellulose, wobei die Hydroxypropylmethylcellulose ein Molekulargewicht von etwa 22.000 Da (g/mol) aufweist und die Hydroxypropylcellulose ein Molekulargewicht von etwa 80.000 Da (g/mol) aufweist, und wobei das Verhältnis der Gewichtsmenge von Saxagliptin in der Glaslösung zu der Gewichtsmenge des Polymers darin 1:2 beträgt.

2. Glaslösung nach Anspruch 1, die ein Röntgenpulverdiffraktogramm mit der Charakteristik eines amorphen Halos aufweist.

3. Glaslösung nach einem der vorangehenden Ansprüche, **dadurch gekennzeichnet, dass** sie eine einphasige Lösung mit einer Glasübergangstemperatur (Tg) von über 100 °C ist.

4. Verfahren zur Herstellung einer Glaslösung von Saxagliptin nach einem der Ansprüche 1-3, wobei Saxagliptinbase, oder ein pharmazeutisch annehmbares Salz davon, und das Polymer in einem Lösungsmittel gelöst werden, welches aus organischen Lösungsmitteln und Wasser, oder Mischungen davon, ausgewählt ist, und dann sprühgetrocknet werden.

5. Verfahren nach Anspruch 4, wobei das organische Lösungsmittel Methanol, Ethanol, Ethylacetat, Isopropanol, Aceton, Dichlormethan, Tetrahydrofuran oder eine Mischung davon ist.

6. Verfahren nach Anspruch 5, wobei das Lösungsmittel ein Gemisch aus DCM und Ethanol ist.

7. Verfahren zur Herstellung einer Glaslösung von Saxagliptin nach einem der Ansprüche 1-3, wobei die Saxagliptinbase, oder ein pharmazeutisch annehmbares Salz davon, mit Polymer gemischt wird und die Mischung unter Verwendung eines temperaturgesteuerten Extruders extrudiert wird.

8. Verwendung der Glaslösung von Saxagliptin in einer Polymermatrix nach einem der vorangehenden Ansprüche für die Herstellung einer pharmazeutischen Zusammensetzung.

## Revendications

1. Solution dite vitreuse à base de la saxagliptine, ou sel en dérivant, acceptable du point de vue pharmaceutique, dispersée dans une matrice polymère, dans laquelle le polymère est choisi parmi l'hydroxypropyl-méthylcellulose et l'hydroxypropylcellulose, dans laquelle l'hydroxypropyl-méthylcellulose présente un poids moléculaire d'environ 22 000 Da (g/mol) et l'hydroxypropylcellulose présente un poids moléculaire d'environ 80 000 Da (g/mol) et dans laquelle le rapport quantité en poids de saxagliptine au sein de la solution vitreuse/quantité en poids du polymère est de 1/2.

2. Solution vitreuse selon la revendication 1, qui présente un diagramme de diffraction des rayons X sur poudre ayant des caractéristiques d'un halo amorphe.

3. Solution vitreuse selon l'une quelconque des revendications précédentes **caractérisée en ce qu'**elle constitue une seule phase ayant une température de transition vitreuse (Tg) supérieure à 100 °C.

4. Un procédé pour la préparation d'une solution vitreuse de saxagliptine, définie selon l'une quelconque des revendications 1 à 3, dans laquelle la base de saxagliptine ou un sel en dérivant, acceptable du point de vue pharmaceutique, et le polymère sont dissous dans un solvant choisi parmi les solvants organiques et l'eau ou de mélange de ceux-ci puis séché par pulvérisation.

5. Le procédé selon la revendication 4, dans lequel le solvant organique est le méthanol, l'éthanol, l'acétate d'éthyle, l'isopropanol, l'acétone, le dichlorométhane, le tétrahydrofuranne ou un mélange de ceux-ci.

6. Le procédé selon la revendication 5, dans lequel le solvant est un mélange de DCM et d'éthanol.

7. Un procédé pour la préparation d'une solution vitreuse de saxagliptine, définie selon l'une quelconque des revendications 1 à 3, dans lequel la base de saxagliptine, ou un sel en dérivant acceptable du point de vue pharmaceutique, est mélangé avec le polymère et le mélange est extrudé en utilisant une extrudeuse à température contrôlée.

8. Utilisation de la solution vitreuse de saxagliptine dans une matrice de polymère selon l'une quelconque des revendications précédentes pour la préparation d'une composition pharmaceutique.
